# EUROPÄISCHE PATENTSCHRIFT

(11) **EP 0 186 019 B1**
(45) Veröffentlichungstag und Bekanntmachung des Hinweises auf die Patenterteilung: **06.10.1993**
(21) Anmeldenummer: 85115594.5
(22) Anmeldetag: 07.12.1985
(51) Int. Cl.: A61L 15/58, A61L 15/44

(54) **Wirkstoffpflaster**
Medicated dressing
Pansement médicamenteux

(30) Priorität: 22.12.1984 DE 3447072
(43) Veröffentlichungstag der Anmeldung: 02.07.1986
(73) Patentinhaber: SCHWARZ PHARMA AG, D-40789 Monheim/Rhld. (DE)
(72) Erfinder: Cordes, Günter, Dr., D-5653 Leichlingen 1 (DE); Wolff, Michael, Dr., D-4019 Monheim (DE)
(74) Vertreter: Werner, Dietrich H., Dr.-Ing.

(56) Entgegenhaltungen:
- EP-A- 0 055 023
- EP-A- 0 072 251
- EP-A- 0 137 278
- GB-A- 2 073 588
- GB-A- 2 095 108

## Beschreibung

Die vorliegende Erfindung betrifft Pflasterformulierungen auf Kautschukbasis zur gesteuerten, über einen längeren Zeitraum anhaltenden transdermalen Applikation von Wirkstoffen insbesondere Beta-Blockern, Steroidhormonen, Calciumantagonisten und herzwirksamen Medikamenten an die Haut mit verbesserten Wirkstofffreisetzungseigenschaften sowie Vorteilen in der Entwicklung und Herstellung der Pflasterfilme.

Zubereitungen, bei denen wasserlösliche Wirkstoffe in einer wasserunlöslichen Matrix dispergiert werden, führen bekanntlich zu einer verzögerten Wirkstofffreisetzung aus der Zubereitung. Die kumulativ pro Zeiteinheit freigesetzten Wirkstoffmengen sind in der Regel sowohl bei homogenen als auch bei heterogenen Matrixsystemen der Quadratwurzel aus der Zeit proportional, wobei das Freigabeprofil u.a. auch von der Geometrie der Darreichungsform abhängig ist. So erhält man beim Übergang vom kugel- zu filmförmigen oder flächigen System einen zunehmend flacheren Verlauf der die Freisetzung des Wirkstoffes pro Zeiteinheit wiedergebenden Kurve, d.h. der oder die Wirkstoffe werden über einen immer längeren Zeitraum in einer praktisch konstanten Rate freigesetzt.

So beschreibt EP-A 0055023 eine klebfähige Zubereitung für antiseptische Mittel, die so hohe Mengen wie 30 - 60 Gew.-% eines elastomeren Materials enthält, das selbst keine fertige Kleberformulierung ist. EP-A 0072251 beschreibt ein hydrophiles Matrixsystem bestehend aus einer festen und einer flüssigen Phase.

Sogenannte Wirkstoffpflaster, bei denen die Wirkstoffe in dünnen, hydrophoben Klebefilmen verteilt sind, stellen demgegenüber konzeptionell einfache, serienmäßig herstellbare pharmazeutische Zubereitungen für Wirkstoffe dar, die prinzipiell zur transdermalen Application von Wirkstoffen geeignet sind.

In der Praxis sind jedoch die aus dem Stand der Technik bislang bekannten Wirkstoffpflaster dieser Art nicht anwendbar für eine kontrollierte transdermale Wirkstoffzufuhr über längere Zeiträume. So reicht die thermodynamische Aktivität des Wirkstoffes in der Pflasterunterlage und in der Haut einschließlich der bisher bekannt gewordenen vorgesehenen Steuerungshilfen nicht aus, um die notwendige Wirkstofffreisetzungsrate und insbesondere auch die gewünschte lange Zeitdauer der Wirkstofffreisetzung bei einer noch akzeptablen Pflastergröße zu erzielen.

Arbeitet man eine größere Wirkstoffmenge in ein solches Pflaster ein als dem Sorptionsvermögen der filmbildenden Pflasterbestandteile entspricht, so muß der Wirkstoff möglichst fein bis amprph in der Klebermatrix verteilt sein, um durch rasches Nachlösen über die Applikationsdauer den Sättigungszustand des Haftklebers weitmöglichst aufrecht zuerhalten und auf diese Weise den Grad der Abnahme der Geschwindigkeit der Freisetzung des Wirkstoffes aus dem Pflaster so klein wie möglich zu halten. Eine Möglichkeit in Bezug auf die geläufigen Ausstreichverfahren zur Herstellung von Pflasterfilmen besteht darin, Filmbildner und Wirkstoff gemeinsam in einem organischen Lösungsmittel zu lösen, die Lösung bis zur streichfähigen Viskosität einzuengen und die wirkstoffhaltige Kleberlösung dann auf großflächige Bahnen auszustreichen und zu trocknen. Dieses Verfahren führt jedoch bei leicht flüchtigen, flüssigen oder kristallinen Wirkstoffen für die bei Kautschuk-Pflasterfilmen üblichen Kleberformulierungen zu Stabilitätsproblemen, bedingt durch Nachkristallisations- und Verdunstungsprozesse, die zu einer unkontrollierten, unreproduzierbaren Wirkstofffreisetzung und Verschlechterung der Klebeeigenschaften führen.

Die galenische Entwicklung von Wirkstoffpflastern dieser Art gestaltet sich darüberhinaus in der Praxis dadurch besonders schwierig, daß die Pflasterrezeptur sowohl bezüglich ihrer Klebeeigenschaften als auch bezüglich ihrer Durchlässigkeit für den oder die eingearbeiteten Wirkstoff bzw. Wirkstoffe zu optimieren ist.

Aufgabe der vorliegenden Erfindung ist es daher die vorgenannten Nachteile bisheriger Wirkstoffpflaster, insbesondere auf dem Gebiet der Betablocker, Steroidhormone, Calciumantagonisten und herzwirksamen Wirkstoffen wie Bupranolol, Propranolol, Estradiol, Nitroglycerin oder Isosorbitdinitrat, sowohl im Hinblick auf ihre Wirkstofffreisetzung als auch ihre Herstellung bzw. Entwicklung zu überwinden und ein Wirkstoffpflaster bereitzustellen, das eine reproduzierbare, über den gesamten Applikationszeitraum möglichst kontrollierte Wirkstoffabgabe in insgesamt hoher Wirkstoffmenge gewährleistet.

Das erfindungsgemäße Wirkstoffpflaster zur kontrollierten Abgabe insbesondere der vorgenannten Wirkstoffe an die Haut besteht wie die vorbekannten Wirkstoffpflaster aus einer undurchlässigen Deckschicht, einem damit verbundenen wasserunlöslichen Klebefilm bestehend aus einer Kautschuk/Klebeharzmasse auf der Basis von Natur- oder Synthesekautschuken wie z.B. Polyisobutylen, Styrol-Butadien-Polymerisaten, Styrol-Isopren-Polymerisaten, Styrol-Ethylen/Butylen-Polymerisaten und cis-1,4-Polyisopren sowie einem Harzanteil, z.B. Kolophonium und dessen Derivate, Polyterpenharze aus β-Pinen, Kohlenwasserstoffharze, in der der bzw. die Wirkstoffe löslich ist bzw. sind und teils in ungelöstem, teils in gelöstem Zustand vorliegt bzw. vorliegen und einer den Klebefilm abdeckenden, wieder ablösbaren Schutzschicht. Das erfindungsgemäße Wirkstoffpflaster ist dadurch gekennzeichnet, daß es, zusammen mit dem bzw. den Wirkstoffen, ein oder mehrere, in Wasser quellbare Polymere aus der Gruppe Galaktomannan, Cellulose und Tragant in einer Menge von 3 bis 30 Gew.-%, bezogen auf die wirkstoffhaltige Kautschuk/Klebeharzmasse, enthält.

Überraschenderweise wird durch den erfindungsgemäßen Einbau der speziellen in Wasser quellfähigen Polymeren als Füllstoffe, die in der Kautschuk/Klebeharzmasse unlöslich sind, die Freisetzungsrate pro Zeiteinheit und insbesondere die Freisetzungsmenge insgesamt um bis zu 100 und mehr Prozent erhöht. Demgegenüber ist aus der Literatur bekannt (vgl. Y.W. Chien in J.R. Robinson "Substained and Controlled Release Drug Delivery Systems", Kap. 4, S. 255-256, Marcel Dekker-Verlag, 1978), daß in der Pflastertechnologie gebräuchliche Füllstoffe wie z.B. Siliciumdioxid und Zinkoxid in polymeren Matrixsystemen auf Silikonbasis bzw. Naturkautschuk zu einer Erniedrigung des Diffusionskoeffizienten von festen und gasförmigen Stoffen führen. Außerdem wird bei der Herstellung durch den Zusatz der speziellen in Wasser quellbaren Polymeren eine stabile Bindung überschüssiger der in der Klebemasse befindlichen Wirkstoffanteile und/oder eine Erhöhung der Viskosität der bei der Fertigung der erfindungsgemäßen Wirkstoffpflaster verwendeten Kleberlösung sowie eine Verbesserung der Kohärenz und Hafteigenschaften des Klebefilms erreicht.

Die im erfindungsgemäßen Wirkstoffpflaster in der Kautschuk/Klebeharzmasse zugesetzten, speziellen, in Wasser quellfähigen Polymeren sind Produkte aus der Gruppe Galaktomannane, Cellulose und Tragant.

Als Cellulose ist besonders bevorzugt mikrokristalline Cellulose. Ganz besonders bevorzugt sind Galaktomannane und Tragant für das Steroidhormon Estradiol, mikrokristalline Cellulose für Bupranolol und Nitroglycerin.

Gemäß einer besonderen Ausführungsform der vorliegenden Erfindung unterteilt sich der Klebefilm aus der Kautschuk/Klebeharzmasse in eine einschichtige Wirkstoff- und das in Wasser quellbare Polymere enthaltende Reservoirschicht aus der Kautschuk/Klebeharzmasse und eine zur Schutzschicht hin liegende, gegebenenfalls Wirkstoff enthaltende Haftklebeschicht aus der Kautschuk/Klebeharzmasse und befindet sich zwischen der Reservoirschicht und der Haftklebeschicht eine Trennschicht, die für die Kautschuk/Klebeharzmasse und den in ihr gelösten Wirkstoff vollständig, für das in Wasser quellbare Polymere nicht oder nur teilweise durchlässig ist. Die Ausführungsform mit der Trennschicht ist bevorzugt, wenn der Klebefilm sich in eine Reservoirschicht und eine Haltklebeschicht unterteilt.

Wie die nachfolgenden Beispiele 1 bis 10 zeigen beeinflussen die erfindungsgemäß eingesetzten, speziellen in Wasser quellfähigen Polymeren in Klebefilmen auf der Basis von Kautschuk/Klebeharzmasse maßgeblich die Freisetzungseigenschaften lipophiler Wirkstoffe. Das therapeutisch gewünschte Freigabeprofil läßt sich für einen gegebenen Wirkstoff vorteilhafterweise durch die Wahl eines der erfindungsgemäß einzusetzenden Produkte aus der Gruppe Galaktomannan, Cellulose und Tragant, die Konzentration an diesen Produkten und die Kombination verschiedener dieser Produkte innerhalb der erfindungsgemäßen Grenzen erhalten, ohne auf eine neue Pflastergrundlage wechseln zu müssen.

Gerade im Fall von Nitroglycerin und Isosorbitdinitrat besteht bei einfachen Klebebändern, in denen der Wirkstoff in gelöster Form vorliegt, nach Literaturangaben die Gefahr einer zu raschen, unkontrollierten Freisetzung sowie von Stabilitäts-, Dosierungs- und Handhabungsproblemen (vgl. z.B. DE-A- 3 200 369). Die therapeutisch erforderliche Menge kann dann nur durch Anwendung großflächiger Pflaster erreicht werden oder die Klebergrundlage vermag den Wirkstoff nicht ausreichend zu binden, so daß die Wirkstofffreigabe in zu kurzer Zeit für eine Langzeitbehandlung erfolgt.

Übliche Herstellverfahren, bei denen die Pflasterkomponenten in einem organischen Lösungsmittel bis zur technisch erforderlichen, ausstreichfähigen Viskosität gelöst bzw. dispergiert werden können dadurch zur Ausbildung instabiler, übersättigter Systeme führen, daß zunächst mehr Wirkstoff gelöst wird als dem Sorptionsvermögen der filmbildenden Bestandteile entspricht. In diesem Fall treten, insbesondere bei leicht flüchtigen Stoffen, bereits beim Trocknungsprozeß erhebliche Gehaltsminderungen auf bzw. der überschüssige Wirkstoffanteil kristallisiert bei Lagerung in dem Klebefilm aus. Diese Probleme werden bei den erfindungsgemäßen Wirkstoffpflastern durch den Einsatz von solchen unlöslichen Produkten umgangen, die den Wirkstoff beim Eindampfen binden können. Hierdurch wird zugleich eine feine Verteilung des Wirkstoffes in der Klebermatrix erreicht, und es ist die Einarbeitung von über die Sättigungslöslichkeit der filmbildenden Bestandteile hinausgehenden Wirkstoffmengen möglich. Durch Ausstreichen der Kleberlösung auf Gewebe, die für die in Wasser quellbaren Polymeren bzw. Wirkstoffadsorbate an diesen Produkten nicht oder nur teilweise durchlässig ist, wird darüberhinaus auf einfachem Weg eine Aufkonzentrierung und Konzentrationsabstufung der wirksamen Bestandteile und/oder der in Wasser quellfähigen Produkte in der Kautschuk/Selbstklebemasse erreicht, falls dies aus klebetechnischen und/oder biopharmazeutischen Gründen erforderlich ist.

Die Verwendung für die Klebemasse durchlässigen Gewebematerialien erspart z.B. das Aufbringen einer zusätzlichen Klebeschicht, die in Abhängigkeit von der Konzentration an den in Wasser quellfähigen Produkten für das Aufbringen der undurchlässigen Deckschicht oder für eine ausreichende Haftung auf der Haut notwendig ist.

Die Steuerungsmöglichkeit der Wirkstofffreisetzung aus Kautschukpflastern durch die in Wasser quellfähigen Polymeren der erfindungsgemäßen Art ist überraschend. Wie die Beispiele 1 und 2 demonstrieren, kann hierdurch z.B. die Freisetzungsgeschwindigkeit erhöht werden, ohne die Wirkstoffkonzentration der Pflaster zu verändern.

### Beispiel 1

Nitroglycerin-Kautschukpflaster gemäß der vorliegenden Erfindung mit mikrokristalliner Cellulose in einer schichtenförmigen Dispersionszone werden wie folgt hergestellt:
Eine nitroglycerin-/cellulosefreie Haftklebemasse bestehend aus
1,018 g Polyisobutylen (mittleres Molekulargewicht von 900.000 bis 1.400.000; Produkt Oppanol B 100®),
0,916 g festem aliphatischem Kohlenwasserstoffharz (Produkt Piccotac® CBHT),
0,916 g hydriertem Colophoniumharz (Produkt Abitol®),
0,094 g Triglycerid (Produkt Miglyol 812®) als Lösungsmittel,
32 g n-Hexan als Lösungsmittel
wird so auf eine einseitig mit Aluminium bedampfte und beidseitig abhäsig ausgerüstete Schutzschicht aufgetragen, daß nach Abdampfen des Lösungsmittels ein Klebefilm von ca. 4,6 mg/cm² erhalten wird. Auf den so hergestellten Haftklebefilm wird die nitroglycerin/cellulosehaltige Dispersionsschicht mit einem Flächengewicht von ca. 19,2 mg/cm² aufkaschiert.

Die Herstellung dieser Schicht erfolgt in analoger Weise aus
2,2671 g Polyisobutylen (mittleres Molekulargewicht von 900.000 bis 1.400.000; Produkt Oppanol B 100®),
2,0409 g festem aliphatischem Kohlenwasserstoffharz (Produkt Piccotac® CBHT),
2,0409 g hydriertem Colophoniumharz (Produkt Abitol®),
0,2071 g Triglycerid (Produkt Myglyol 812®) als Lösungsmittel,
5,700 g 5 %-iger (G/G)Nitroglycerin-Cellulose-Verreibung (Produkt 5 % Nitroglycerin/Avicel® pH 105 - Verreibung)
38 g n-Hexan.

Nach Abdecken der Dispersionsschicht mit einer undurchlässigen Deckschicht wird der erhaltene Pflasterfilm den therapeutischen Erfordernissen entsprechend in Einzelstücke aufgeteilt.

### Beispiel 2 (Vergleichsbeispiel)

Die Herstellung erfolgt wie im Beispiel 1, jedoch mit einer 5 % Nitroglycerin-Lactose-Verreibung anstelle der Nitroglycerin-Cellulose-Verreibung.

### Wirkstofffreisetzung:

16 cm² große Pflasterfilme, hergestellt nach den Beispielen 1 und 2, werden in isotonischer Natriumchlorid-Lösung von 37°C eingetaucht und die freigesetzte Menge an Nitroglycerin nach 2, 4, 6, 8 und 24 Stunden flüssigkeitschromatographisch bestimmt. Das Volumen des Freigabemediums wird so gewählt, daß über die Dauer des Versuchs "Sink-Bedingungen" eingehalten werden.

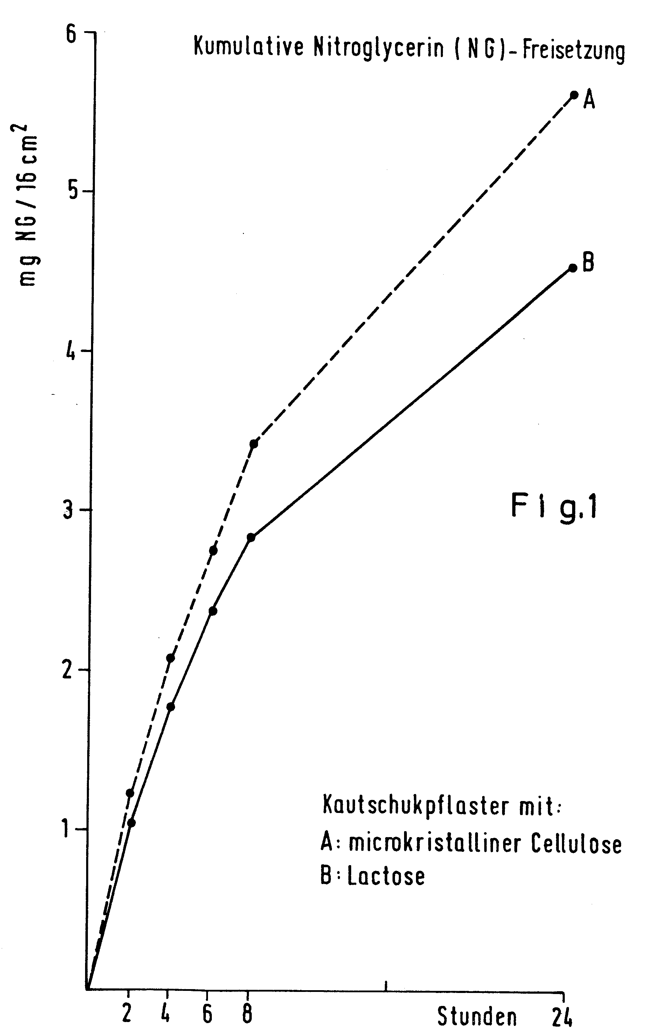

Das Ergebnis wird in Fig. 1 wiedergegeben.

### Beispiel 3

Ein Estradiol als Wirkstoff enthaltendes Pflaster mit Galaktomannan (Produkt Meyprogst 90®) als in Wasser quellfähiges Polymeres in einer haftklebenden Dispersionsschicht wurde wie folgt hergestellt:
Die Estradiol-haltige Haftklebemasse, bestehend aus den in Tabelle 1 angegebenen Bestandteilen und Lösungsmittelanteilen (siehe Rezepturen A, B, C, D), wird so auf eine einseitig mit Aluminium bedampfte und beidseitig abhäsiv ausgerüstete Schutzschicht aufgetragen, daß nach dem Abdampfen des Lösungsmittels ein Klebefilm mit den in Tabelle 1 angegebenen Flächengewichten erhalten wird.

Nach Abdecken der Estradiol/Galaktomannan enthaltenden Haftklebeschicht mit einer undurchlässigen Deckschicht werden die Pflasterfilme den therapeutischen Erfordernissen entsprechend in Einzelstücke aufgeteilt.

**Tabelle 1**

| Herstellformel galaktomannanhaltiger Estradiolpflaster | | | | |
|---|---|---|---|---|
| Bestandteil | Menge (g/1.000 cm²) | | | |
| | Rezeptur | | | |
| | A | B | C | D |
| 17 β-Estradiol mikron. (Teilchengröße < 9 µm) | 0,42 | 0,3 | 0,27 | 0,5 |
| Galaktomannan (Produkt Meyprogat 90®) | 1,5 | 0,9 | 0,9 | 0,5 |
| Polyisobutylen (Produkt Oppanol B 100®) | 3,0 | - | - | - |
| Hydriertes Colophonium (Produkt Abitol®) | 3,0 | - | 1,5 | - |
| Festes hydriertes Kohlenwasserstoffharz (Produkt Piccotac CBHT®) | 3,0 | - | - | - |
| Dreiblock-Polystyrol-PolyisoprenPolystyrol-Copolymer (Produkt Cariflex TR 1107®) | - | 1,7 | 1,7 | 1,7 |
| Festes aromatisches Kohlenwasserstoffharz (Produkt Piccovar L 60®) | - | 2,2 | 2,2 | 2,2 |
| Polyterpen-Harz (Produkt Dercolyte S 10®) | - | 1,5 | - | 1,5 |
| 1,2-Propandiol | 0,5 | 0,15 | 0,15 | 0,2 |
| Triglycerid (Produkt Miglyol 812®) | 0,5 | - | - | - |
| Spezialbenzin 80 - 100 als Lösungsmittel | 64 | 19 | 19 | 19 |
| Flächengewicht mg/cm² | 11,9 | 6,75 | 6,72 | 6,60 |

Zu Vergleichszwecken wurden entsprechend den Rezeptoren A bis C analog zusammengesetzte Estradiol-Pflaster, jedoch ohne Zusatz von Galaktomannan, hergestellt. Tabelle 2 gibt die Bestandteile und Flächengewichte dieser füllstofffreien Klebefilme wieder. Die Abweichung in der quantitativen Zusammensetzung gegenüber den in Tabelle 1 aufgeführten Estradiol-Pflastern ist zum Teil technologisch, zum Teil iopharmazeutisch bedingt; die füllstofffreien Klebefilme wurden im Galenikmaßstab mit dem Ziel entwickelt, eine möglichst hohe Wirkstofffreisetzung zu erreichen.

**Tabelle 2**

| Zusammensetzung galaktomannan-freier Estradiolpflaster | | | |
|---|---|---|---|
| Bestandteil | Menge (g/1.000 cm² | | |
| | Rezeptur | | |
| | A' | B' | C' |
| 17 β-Estradiol mikron. (Teilchengröße < 9 µm) | 0,39 | 0,51 | 0,51 |
| Polyisobutylen (Produkt Oppanol B 100®) | 1,95 | - | - |
| Hydriertes Colophonium (Produkt Abitol®) | 2,34 | - | 1,19 |
| Festes hydriertes Kohlenwasserstoffharz (Produkt Piccotac CBHT®) | 2,34 | - | - |
| Dreiblock-Polystyrol-Polyisopren-Polystyrol-Copolymer (Produkt Cariflex TR 1107®) | - | 1,32 | 1,32 |
| Festes aromatisches Kohlenwasserstoffharz (Produkt Piccovar L 60®) | - | 1,98 | 1,98 |
| Polyterpen-Harz (Produkt Dercolyte S 10®) | - | 1,19 | - |
| 1,2-Propandiol | 0,39 | 0,10 | 0,10 |
| Triglycerid (Produkt Miglyol 812®) | 0,39 | - | - |
| Flächengewicht mg/cm² | 7,8 | 5,1 | 5,1 |

### Wirkstofffreisetzung in vitro:

Die Bestimmung erfolgt wie im Beispiel 1 bzw. 2 beschrieben unter Verwendung von 10 cm² großen Pflasterstücken und bei einer Temperatur von 34°C. Die kumulativen Freisetzungsraten der erfindungsgemäßen Pflaster A bis D und der galaktomannan-freien, ansonsten qualitativ gleich zusammengesetzter Vergleichspflaster A' bis C' sind in Tabelle 3 gegenübergestellt.

Wie zu sehen, bewirkt der Zusatz von Galaktomannan als in Wasser quellfähiges Polymeres in einschichtig aufgebauten Estradiol-Pflasterfilmen eine Erhöhung der kumulativen Estradiol (= E₂)-Freisetzungsraten.

**Tabelle 3**

| E₂-Freisetzung (µg/10 cm²), n = 2 | | | | | | |
|---|---|---|---|---|---|---|
| Zeit (h) | Rezepturen | | | | | |
| | A | A' | B | B' | C | C' |
| 2 | 47,4 | 17,5 | 42,7 | 35,3 | 70,1 | 36,6 |
| 4 | 79,1 | 33,7 | ./. | ./. | ./. | ./. |
| 6 | 108,2 | ./. | ./. | ./. | ./. | ./. |
| 8 | ./. | 55,4 | ./. | ./. | ./. | ./. |
| 24 | 252,1 | 123,4 | 323,3 | 182,1 | 395,4 | 229,9 |

### Wirkstofffreisetzung in vivo:

2 Probanden wurden auf dem seitlichen Brustkorb
a) 1 Pflaster a' 10 cm², hergestellt nach Beispiel 3, Rezeptor B
b) 2 Pflaster a' 10 cm², hergestellt nach Beispiel 3, Rezeptor D

appliziert. Nach 72 Stunden wurden die Pflaster abgezogen und der jeweils darin verbleibende Estradiolgehalt chromatographisch bestimmt. Die so geschätzten Freisetzungsraten, bezogen auf 1 Pflaster a' 10 cm², betrugen:
a) 203 µg - Proband 1 / Rezeptur B
b) 208 µg - Proband 1 / Rezeptur D
c) 92,5 µg - Proband 2 / Rezeptur B
d) 110 µg - Proband 2 / Rezeptur D.

### Bioverfügbarkeit:

Bei den oben geschilderten Probandenversuchen worden 48, 38, 24, 14 Stunden sowie unmittelbar vor der Pflasterapplikation Blutproben entnommen und die Estradiolplasmakonzentrationen radioimmunologisch bestimmt. Nach Aufkleben der Pflaster erfolgen Blutentnahmen nach 10, 24, 34, 48, 58 und 72 Stunden. Im Mittel ergaben sich folgende Anstiege der Estradiolplasmakonzentrationen (Angaben bezogen auf ein Pflaster von 10 cm²):
a) 6,45 pg/ml - Proband 1 / Rezeptur B
b) 6,65 pg/ml - Proband 1 / Rezeptur D
c) 3,90 pg/ml - Proband 2 / Rezeptur B
d) 1,47 pg/ml - Proband 2 / Rezeptur D.

Danach führte die Applikation der Estradiol (E₂)-Pflaster zu einem Anstieg der E₂-Plasmakonzentration beim Mann, wobei die Rezeptor B mit gegenüber Rezeptur D aus Beispiel 3 verringerter E₂-Menge, aber erhöhter Galaktomannankonzentration bei einer Versuchsperson einen vergleichsweise höheren, mittleren E₂-Konzentrationsanstieg im Blut ergab.

### Beispiel 4

Ein Pflaster für Bupranolol als Wirkstoff gemäß der vorliegende Erfindung mit mikrokristalliner Cellulose (Produkt Avicel pH 105®) als in Wasser quellfähigem Polymeren in einer haftklebenden Dispersionsschicht wird wie folgt hergestellt:
Die Bupranolol-haltige Kautschuk/Klebeharzmasse, bestehend aus den in Tabelle 4 angegebenen Bestandteilen (Rezeptur A), wird so in zwei aufeinanderfolgenden Schritten auf eine einseitig mit Aluminium bedampfte und beidseitig abhäsig ausgerüstete Schutzschicht aufgetragen, daß nach Entfernen des Lösungsmittels ein Klebefilm mit einem Flächengewicht von 14,7 mg/cm² resultiert.

Nach Abdecken der Bupranolol/Cellulose-enthaltenden Dispersionsschicht mit einer undurchlassigen Deckschicht werden die Pflasterfilme den therapeutischen Erfordernissen entsprechend in Einzelstücke aufgeteilt.

### Beispiel 5 (Vergleichsbeispiel)

Die Herstellung erfolgt wie in Beispiel 4, jedoch ohne die Verwendung von mikrokristalliner Cellulose als in Wasser quellfähigem Polymeren. Das Flächengewicht des Pflasterfilms beträgt 13,5 mg/cm². Zusammensetzung und Lösungsmittelmenge der Haftklebemasse beim Ausstreichen sind in Tabelle 4 angegeben.

**Tabelle 4**

| Zusammensetzung von Bupranolol-Pflaster mit und ohne mikrokristalliner Cellulose als in Wasser quellfähigem Polymeren (Beispiel 4 und 5) | | |
|---|---|---|
| Bestandteil | Menge (g/1.000 cm²) | |
| | Rezeptur | |
| | A mit Polymeren | B ohne Polymeren |
| Bupranolol mikron. (Teilchengröße < 50 µm) | 1,2 | 1,2 |
| mikrokristalline Cellulose | 1,2 | - |
| Polyisobutylen (Produkt Oppanol B 100®) | 3,71 | 3,71 |
| Festes aromatisches Kohlenwasserstoffharz (Produkt Piccovar L 60®) | 6,69 | 6,69 |
| 1,2-Propandiol | 0,5 | 0,5 |
| Hartparaffin | 1,4 | 1,4 |
| Spezialbenzin 80 - 110 | 55 | 55 |

### Wirkstofffreisetzung:

Die Bestimmung erfolgt wie in Beispiel 1 für die Nitroglycerinpflaster angegeben. Die kumulativen Freisetzungsraten des erfindungsgemäßen Bopranolol-Pflasters gemäß Beispiel 4 und des Cellulose-freien Pflasters gemäß Beispiel 5 sind in Tabelle 5 gegenübergestellt.

Wie zu sehen, bewirkt der Zusatz von Cellulose als in Wasser quellfähigen Polymeren zu einschichtig aufgebauten Bupranolol-Pflasterfilmen eine Erhöhung der kumulativen Wirkstofffreisetzungsraten.

**Tabelle 5**

| Bupranolol-Freisetzung (mg/25 cm²), n = 2 | | |
|---|---|---|
| Zeit | Beispiel 4 | Beispiel 5 |
| 2 | 6,46 | 4,04 |
| 4 | 10,04 | 5,77 |
| 8 | 15,37 | 8,21 |
| 24 | 26,95 | 14,20 |

### Beispiel 6

Estradiol-Pflaster auf Kautschuk-Basis mit in Wasser unterschiedlich stark quellenden Polymeren wurden wie folgt hergestellt:
Die Estradiol-haltige Haftklebemasse, bestehend aus den in Tabelle 6 angegebenen Bestandteilen und Lösungsmittelanteilen (siehe Rezepturen A, B, C), wird so auf eine einseitig mit aluminiumbedampfte und beidseitig abhäsiv ausgerüstete Schutzschicht aufgetragen, daß nach dem Abdampfen des Lösungsmittels ein Klebefilm mit den in Tabelle 6 angegebenen Flächengewichten erhalten wird.

Nach Abdecken der Estradiol/Quellstoff-enthaltenden Haftklebeschicht mit einer undurchlässigen Deckschicht werden die Pflasterfilme den therapeutischen Erfordernissen entsprechend in Einzelstücke aufgeteilt.

**Tabelle 6**

| Zusammensetzung von Estradiol-Pflastern mit unterschiedlichen, in Wasser quellfähigen Polymeren | | | |
|---|---|---|---|
| Bestandteil | Menge (g/3.000 cm²) | | |
| | Rezeptur | | |
| | A | B | C |
| 17 β-Estradiol mikron. (Teilchengröße < 9 µm) | 1,5 | 1,5 | 1,5 |
| Galaktomannan (Produkt Meyprogat 90®) | 1,5 | - | - |
| Tragant | - | 1,5 | - |
| mikrokristalline Cellulose (Produkt Avicel pH 105®) | - | - | 1,5 |
| Dreiblock-Polystyrol-Polyisopren-Polystyrol-Copolymer (Produkt Cariflex TR 1107®) | 5,1 | 5,1 | 5,1 |
| Festes hydriertes Kohlenwasserstoffharz (Produkt Piccotac CBHT®) | 6,6 | 6,6 | 6,6 |
| Polyterpen-Harz (Produkt Dercolyte S 10®) | 4,5 | 4,5 | 4,5 |
| 1,2-Propandiol | 0,6 | 0,6 | 0,6 |
| Spezialbenzin 80 - 100 | 27 | 27 | 27 |
| Flächengewicht mg/cm² | 6,6 | 6,6 | 6,6 |

Tabelle 7 zeigt den zeitlichen Verlauf der Wasseraufnahme der Polymerprodukte in gesättigter Wasserdampfatmosphäre in Prozent des Probengewichtes bei Raumtemperatur.

**Tabelle 7**

| Prüfsubstanz | Wasseraufnahme innerhalb | | |
|---|---|---|---|
| | 48 h | 96 h | 168 h |
| Galaktomannan (Produkt Meyprogat 90®) | 69,72 | 97,86 | 112,14 |
| Tragant | 68,08 | 84,51 | 96,7 |
| mikrokristalline Cellulose (Produkt Avicel pH 105®) | 14,7 | 16,7 | 21,5 |

### Wirkstofffreisetzung:

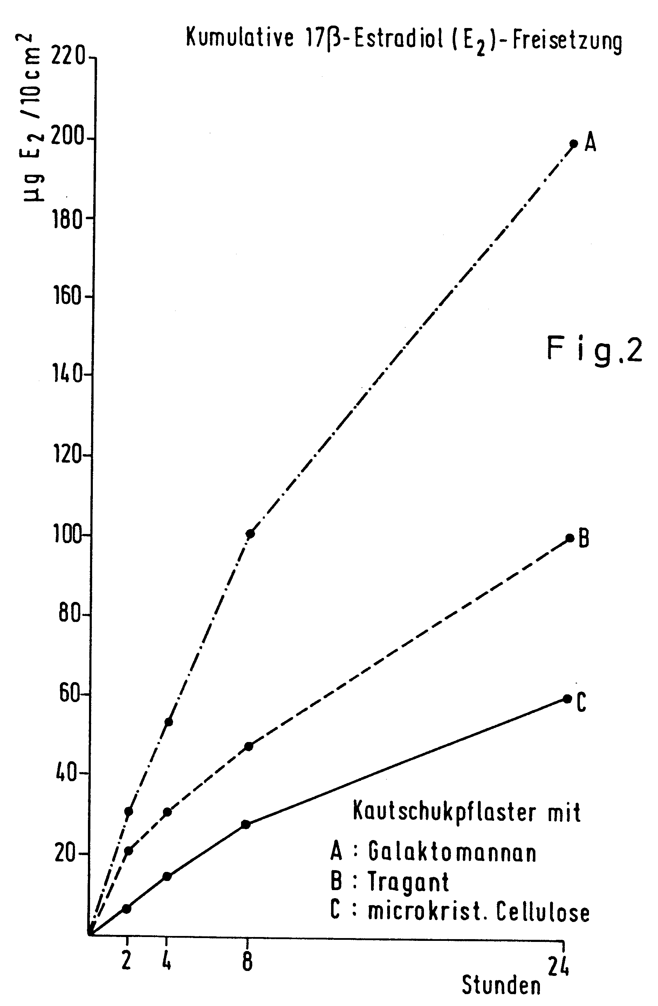

Fig. 2 zeigt den zeitlichen Verlauf der Estradiol-Pflaster 6 A bis 6 E. Die Bestimmung erfolgte wie in Beispiel 1 beschrieben unter Verwendung von 5 cm² großen Pflasterfilmen sowie bei einer Temperatur von 34°C. Die Abbildung zeigt deutlich die Abhängigkeit der Wirkstofffreisetzung von der Art des als Füllstoff eingesetzten, in Wasser quellfähigen Polymers.

Der Vergleich mit Tabelle 7 zeigt, daß die Wirkstofffreisetzung mit zunehmender Wasseraufnahmefähigkeit der eingesetzten Polymerprodukte ansteigt.

### Beispiel 7

Propranolol-Pflaster mit mikrokristalliner Cellulose als in Wasser quellfähigem Polymerprodukt wurden wie folgt hergestellt:
Die propranololhaltige Haftklebemasse bestehend aus

| | |
|---|---|
| Propranolol mikron | 1,2 g |
| mikrokristalline Cellulose (Produkt Avicel pH 105®) | 1,2 g |
| Dreiblock-Polystyrol-Poly(ethylenbutylen)-Polystyrol-Copolymer (Produkt Kraton G 1657®) | 3,71 g |
| Festes aromatisches Kohlenwasserstoffharz (Produkt Piccovar L 60®) | 5,0 g |
| Flüssiges Kohlenwasserstoffgemisch (Produkt Ondinaöl G 33®) | 1,7 g |
| 1,2-Propandiol | 0,34 g |
| Spezialbenzin 80 - 100 | 30 g |

wird in zwei aufeinanderfolgenden Teilschritten so auf eine einseitig mit Aluminium bedampfte und beidseitig abhäsiv ausgerüstete Schutzschicht aufgetragen, daß nach Entfernen des Lösemittels eine Schicht von ca. 13,1 mg/cm² erhalten wird. Nach Abdecken der Haftklebeschicht mit einer undurchlässigen Deckschicht wird der Pflasterfilm den therapeutischen Erfordernissen entsprechend in Einzelstücke aufgeteilt.

### Wirkstofffreisetzung:

Die Bestimmung erfolgt wie im Beispiel 1 angegebenen bei 34°C, die kumulativen Freisetzungsraten des erfindungsgemäßen Propranolol-Pflasters gemäß Beispiel 7 betrugen 5,64; 11,31; 20,0 und 26,79 mg/25 cm² nach 2, 4, 8 bzw. 24 Stunden (Mittelwerte aus zwei Bestimmungen).

### Beispiel 8

Ein Verapamil als Wirkstoff enthaltendes Pflaster mit Galaktomannan (Produkt Meyprogat 90®) als in Wasser quellfähiges Polymeres in einer schichtenförmigen Dispersionszone wird wie folgt hergestellt:
Eine galaktomannanfreie Haftklebemasse bestehend aus
1,08 g Polyisobutylen (mittleres Molekulargewicht von 900.000 bis 1.400.000) (Produkt Oppanol B 100®)
1,35 g festem aromatischen Kohlenwasserstoffharz (Produkt Piccovar L 60®)
0,96 g Polyterpen-Harz (Produkt Dercolyte S 10®)
0,24 g Polyethylenglykol (mittleres Molekulargewicht von 300) (Produkt Lutrol 300®)
0,3 g Verapamil/Siliciumdioxid-Mischung 1:1 (Produkt Aerosil 200®)
10 g Spezialbenzin 80 - 110 als Lösungsmittel
wird so auf eine einseitig mit Aluminium bedampfte und beidseitig abhäsiv ausgerüstete Schutzschicht aufgetragen, daß nach Abdampfen des Lösungsmittels ein Klebefilm von ca. 1,3 mg/cm² erhalten wird. Auf den so hergestellten Haftklebefilm wird die galaktomannanhaltige Dispersionsschicht mit einem Flächengewicht von ca. 16,6 mg/cm² aufkaschiert. Die Herstellung dieser Schicht erfolgt in analoger Weise aus
10,8 g Polyisobutylen (mittleres Molekulargewicht von 900.000 bis 1.400.000) (Produkt Oppanol B 100®)
13,5 g festem aromatischem Kohlenwasserstoffharz (Produkt Piccobar L 60®)
9,6 g Polyterpen-Harz (Produkt Dercolyte S 10®)
2,4 g Polyethylenglykol (mittleres Molekulargewicht von 300; Produkt Lutrol 300®)
1,5 g Galaktomannan (Produkt Meyprogat 90®)
12,0 g Verapamil/Siliciumdioxid-Mischung 1:1 (Produkt Aerosil 200®)
100,0 g Spezialbenzin 80 - 110

wobei der Auftrag auf eine undurchlassige Deckschicht erfolgt. Der erhaltene Pflasterfilm wird den therapeutischen Erfordernissen entsprechend in Einzelstücke aufgeteilt.

### Beispiel 9 (Vergleichsbeispiel)

Die Herstellung erfolgt entsprechend Beispiel 8, jedoch ohne Galaktomannan als in Wasser quellfähigem Polymeren.

### Wirkstofffreisetzung in vivo:

Einem Probanden wurde auf den Unterarm (Innenseite) je ein 5 cm² großes, nach Beispiel 8 bzw. 9 hergestelltes pflaster aufgeklebt. Nach 24 Stunden wurden die Pflaster abgezogen und der jeweils darin verbliebene Verapamilgehalt chromatographisch bestimmt.

Die freigesetzten Verapamilmengen betrugen
a) für Beispiel 8 (mit Galaktomannan): 0,31 mg/cm²
b) für Beispiel 9 (ohne Galaktomannan): 0,15 mg/cm²
Dieses Ergebnis zeigt, daß durch Einsatz quellfähiger Polymerer bei sonsti identischer Pflasterrezeptur eine Verdoppelung der in vivo-Verapamilfreisetzungsrate erreicht werden konnte.

### Beispiel 10

Ein Pupranolol als Wirkstoff enthaltendes Pflaster mit mikrokristalliner Cellulose (Produkt Avicel pH 105®) als in Wasser quellfähiges Polymeres in einer haftklebenden Dispersionsschicht wird wie folgt hergestellt:
Eine bupranololhaltige Kautschuk/Klebeharzmasse bestehend aus

| | |
|---|---|
| Bupranolol | 6,0 g |
| Mikrokristalline Cellulose (Produkt Avicel pH 105®) | 6,0 g |
| Polyisobutylen (Produkt Oppanol B 100®) | 18,55 g |
| Festes aromatisches Kohlenwasserstoffharz (Produkt Piccorvar L 60®) | 33,45 g |
| Flüssiges Kohlenwasserstoffgemisch (Produkt Ondinaöl G 33®) | 11,1 g |
| 1,2 -Propandiol | 1,7 g |
| Spezialbenzin 80 - 110 | 191,15 g |

wird so auf eine einseitig mit Aluminium bedampfte und beidseitig abhäsiv ausgerüstete Schutzschicht aufgetragen, daß nach Entfernen des Lösungsmittels ein Klebefilm mit einem Flächengewicht von ca. 15,4 mg/cm² resultiert. Nach Abdecken mit einer undurchlässigen Deckschicht werden die Pflasterfilme den therapeutischen Erfordernissen entsprechend in Einzelstücke aufgeteilt.

### Wirkstofffreisetzung in vitro:

Die Bestimmung erfolgte wie in Beispiel 1 beschrieben an 16 cm² Pflasterfilmen in Phosphatpufferlösung (pH = 5,5) als Freisetzungsmedium und bei 34°C. Die kumulativ freigesetzten Wirkstoffmengen betrugen nach 2, 4, 8 und 24 Stunden 3,12; 4,30; 5,89 bzw. 10,44 mg.

### Wirkstofffreisetzung in vivo:

6 Probanden wurde auf dem seitlichen Brustkorb über einen Zeitraum von 3 Tagen alle 24 Stunden je ein 25 cm² großes, nach Beispiel 10 hergestelltes Pflaster aufgeklebt. Jeweils nach 24 Stunden wurden die Pflaster abgezogen und der verbliebene Bupranololgehalt chromatographisch bestimmt. Die Mittelwerte der individuell freigesetzten Wirkstoffmengen betrugen 13,49; 11,25; 13,70; 10,44; 14,76 bzw. 12,81 mg/25 cm²/24 Stunden.

Interindividuell wurden im Mittel 12,74 ± 1,84 mg (n = 3 x 6) Bupranolol pro Pflaster freigesetzt.

In diesem Versuch zeigte sich eine gute Reproduzierbarkeit in vivo-Freisetzung und eine relativ gute Übereinstimmung mit dem entsprechenden in vitro-Ergebnis von 16,31 mg/25 cm²/24 Std. (≙ 10,44 mg/25 cm²/24 Std., s.o.).

### Bioverfügbarkeit:

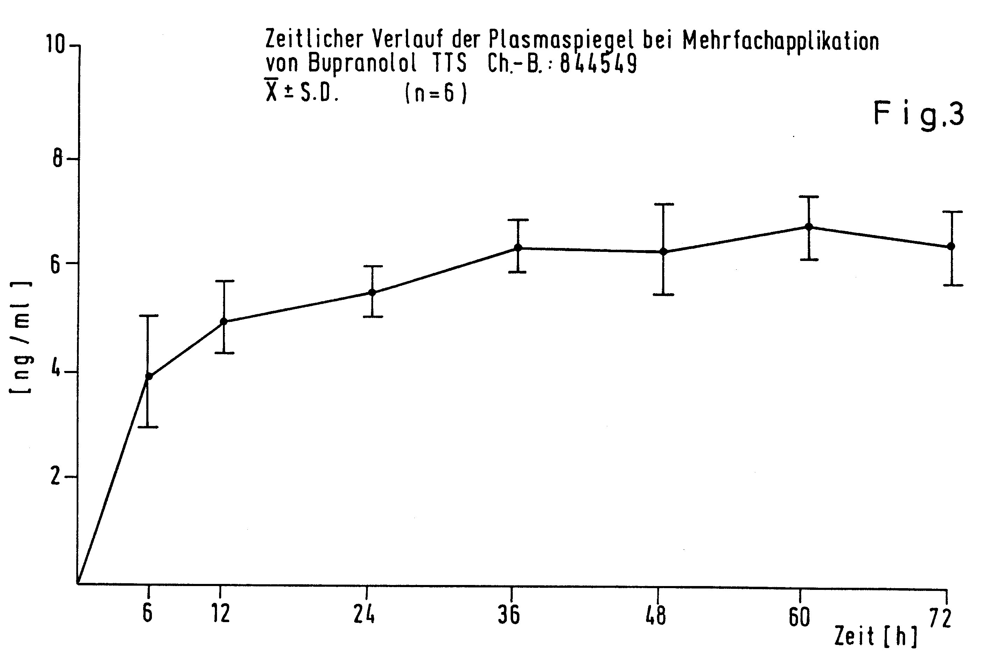

Bei dem oben geschilderten Probandenversuch wurden nach Applikationszeiten von 6, 12, 24, 36, 48, 60 und 72 Stunden Blutproben entommen und die Bupranololplasmakonzentration radioimmunologisch bestimmt. Die Ergebnisse sind in Figur 3 wiedergegeben.

Entsprechend kontinuierlicher Wirkstofffreisetzung wird im Applikationszeitraum von 3 Tagen ein konstanter Plasmaspiegel aufgebaut.

## Patentansprüche (Patentansprüche für folgende(n) Vertragsstaat(en): BE, CH, DE, FR, GB, IT, LI, LU, NL, SE)

1. Wirkstoffpflaster zur kontrollierten Abgabe von Arzneistoffen an die Haut, bestehend aus einer Deckschicht, einem damit verbundenen, wasserunlöslichen Klebefilm aus einer Kautschuk/Klebeharzmasse, in der der bzw. die Wirkstoffe löslich oder teilweise löslich ist bzw. sind mit einem in Wasser quellbaren, in der Kautschuk/Klebemasse unlöslichen Zusatzstoff, und einer den Klebefilm abdeckenden, wieder ablösbaren Schutzschicht, dadurch gekannzeichnet, daß man als Zusatzstoff eine Verbindung aus der Gruppe Galaktomannan, Cellulose und Tragant in einer Menge von 3 bis 30 Gew.-%, bezogen auf das Gewicht der Kautschuk/Klebeharzmasse verwendet.

2. Wirkstoffpflaster gemäß Anspruch 1, dadurch gekennzeichnet, daß als Arzneistoffe β-Blocker-Steroidhormone, Calciumantagonisten und herzwirksame Medikamente verwendet werden.

3. Wirkstoffpflaster gemäß einem der Ansprüche 1 oder 2, dadurch gekennzeichnet, daß die Kautschuk/Klebeharzmasse unterteilt ist zwischen einer Wirkstoff und das in Wasser quellbare Polymere enthaltenden Reservorischicht und einer gegebenenfalls Wirkstoff enthaltenden Haftklebeschicht, wobei zwischen der Reservoirschicht und der Haftklebeschicht eine Trennschicht vorgesehen ist, die für die Kautschuk/Klebeharzmasse und darin gelöstem Wirkstoff durchlässig, für das in Wasser quellbare Polymere völlig oder im wesentlichen undurchlässig ist.

4. Wirkstoffpflaster für Estradiol gemäß einem der Ansprüche 1 bis 3, dadurch gekennzeichnet, daß das in Wasser quellbare Polymere ein Galaktomannan ist.

5. Wirkstoffpflaster für Bupranolol gemäß einem der Ansprüche 1 bis 3, dadurch gekennzeichnet, daß das in Wasser quellbare Polymere mikrokristalline Cellulose ist.

6. Wirkstoffpflaster für Nitroglycerin gemäß einem der Ansprüche 1 bis 3, dadurch gekennzeichnet, daß das in Wasser quellbare Polymere mikrokristalline Cellulose ist.

7. Wirkstoffpflaster für Propranolol gemäß einem der Ansprüche 1 bis 3, dadurch gekennzeichnet, daß das in Wasser quellbare Polymere mikrokristalline Cellulose ist.

8. Verfahren zur Herstellung von Wirkstoffpflastern gemäß Ansprüchen 1 bis 7, dadurch gekennzeichnet, daß man die Bestandteile der Kautschuk/Klebemasse in einem organischen Lösungsmittel löst und in der Lösung den Wirkstoff zusammen mit dem Zusatzstoff in einer Menge von 3 bis 30 Gew.-%, bezogen auf das Gewicht der Kautschuk/Klebeharzmasse, dispergiert, die Dispersion, gegebenenfalls nach Verdampfen eines Teils des Lösungsmittels, auf einer adhasiv ausgerüsteten Schutzschicht aufträgt, das Lösungsmittel im wesentlichen verdampft, eine Deckschicht aufträgt und den so erhaltenen Pflasterfilm in Teile von therapeutisch notwendiger Größe zerteilt.

9. Verfahren gemäß einem der Ansprüche 8 bis 10, dadurch gekennzeichnet, daß der Wirkstoff Estradiol und der Zusatzstoff ein Galaktomannan ist.

10. Verfahren gemäß Anspruch 8, dadurch gekennzeichnet, daß der Wirkstoff Bupranolol und der Zusatzstoff mikrokristalline Cellulose ist.

11. Verfahren gemäß Anspruch 8, dadurch gekennzeichnet, daß der Wirkstoff Nitroglycerin und der Zusatzstoff mikrokristalline Cellulose ist.

12. Verfahren gemäß Anspruch 8, dadurch gekennzeichnet, daß der Wirkstoff Propranolol und der Zusatzstoff mikrokristalline Cellulose ist.

## Patentansprüche (Patentansprüche für folgende(n) Vertragsstaat(en): AT)

1. Verfahren zur Herstellung von Wirkstoffpflastern zur kontrollierten Abgabe von Arzneistoffen an die Haut, bestehend aus einer Deckschicht, einem damit verbundenen, wasserunlöslichen Klebefilm aus einer Kautschuk/Klebeharzmasse, in der der bzw. die Wirkstoffe löslich oder teilweise löslich ist bzw. sind mit einem in Wasser quellbaren, in der Kautschuk/Klebemasse unlöslichen Zusatzstoff, und einer den Klebefilm abdeckenden, wieder ablösbaren Schutzschicht, dadurch gekennzeichnet, daß man die Bestandteile der Kautschuk/Klebemasse in einem organischen Lösungsmittel löst und in der Lösung den Wirkstoff zusammen mit einer Verbindung aus der Gruppe Galaktomannan, Cellulose und Tragant als Zusatzstoff in einer Menge von 3 bis 30 Gew.-%, bezogen auf das Gewicht der Kautschuk/Klebeharzmasse, dispergiert, die Dispersion, gegebenenfalls nach Verdampfen eines Teils des Lösungsmittels, auf einer adhäsiv ausgerüsteten Schutzschicht aufträgt, das Lösungsmittel im wesentlichen verdampft, eine Deckschicht aufträgt und den so erhaltenen Pflasterfilm in Teile von therapeutisch notwendiger Größe zerteilt.

2. Verfahren gemäß Anspruch 1, dadurch gekennzeichnet, daß als Arzneistoffe β-Blocker-Steroidhormone, Calciumantagonisten und herzwirksame Medikamente verwendet werden.

3. Verfahren gemäß einem der Ansprüche 1 oder 2, dadurch gekennzeichnet, daß man die Kautschuk/Klebeharzmasse unterteilt in einen Wirkstoff und den Zusatzstoff enthaltende Reservorischicht und eine gegebenenfalls Wirkstoff enthaltende Haftklebeschicht, wobei zwischen der Reservoirschicht und der Haftklebeschicht eine Trennschicht vorgesehen wird, die für die Kautschuk/Klebeharzmasse und darin gelöstem Wirkstoff durchlässig, für den Zusatzstoff völlig oder im wesentlichen undurchlässig ist.

4. Verfahren gemäß einem der Ansprüche 1 bis 3, dadurch gekennzeichnet, daß der Wirkstoff Estradiol und der Zusatzstoff ein Galaktomannan ist.

5. Verfahren gemäß einem der Ansprüche 1 bis 3, dadurch gekennzeichnet, daß der Wirkstoff Bupranolol und der Zusatzstoff mikrokristalline Cellulose ist.

6. Verfahren gemäß einem der Ansprüche 1 bis 3, dadurch gekennzeichnet, daß der Wirkstoff Nitroglycerin und der Zusatzstoff mikrokristalline Cellulose ist.

7. Verfahren gemäß einem der Ansprüche 1 bis 3, dadurch gekennzeichnet, daß der Wirkstoff Propranolol und der Zusatzstoff mikrokristalline Cellulose ist.

## Claims (Claims for the following Contracting State(s): BE, CH, DE, FR, GB, IT, LI, LU, NL, SE)

1. A medicated dressing for the controlled release of drugs to the skin, comprising a covering layer, a water-insoluble adhesive film of a rubber/adhesive resin substance which is connected thereto and in which the or each drug is soluble or partially soluble and which contains an additive substance swellable in water and insoluble in the rubber/adhesive substance, and a releasable protective layer which covers the adhesive film, characterized in that the additive substance used is a compound selected from the group formed by galactomannan, cellulose and tragacanth in a quantity of 3 to 30% by weight, referred to the weight of the rubber/adhesive resin substance.

2. A medicated dressing according to claim 1, characterized in that the drugs used are beta blockers, steroid hormones, calcium antagonists and cardiac-acting medicaments.

3. A medicated dressing according to one of claims 1 or 2, characterized in that the rubber/adhesive resin substance is subdivided between a reservoir layer containing drug and the polymer swellable in water and an adhesive layer possibly containing drug, while provided between the reservoir layer and the adhesive layer is a separating layer which is permeable to the rubber/adhesive resin substance and drug dissolved therein, but is completely or substantially impermeable to the polymer swellable in water.

4. A medicated dressing for estradiol according to one of claims 1 to 3, characterized in that the polymer swellable in water is a galactomannan.

5. A medicated dressing for bupranolol according to one of claims 1 to 3, characterized in that the polymer swellable in water is microcrystalline cellulose.

6. A medicated dressing for nitroglycerine according to one of claims 1 to 3, characterized in that the polymer swellable in water is microcrystalline cellulose.

7. A medicated dressing for propranolol according to one of claims 1 to 3, characterized in that the polymer swellable in water is microcrystalline cellulose.

8. A process for the preparation of medicated dressings according to claims 1 to 7, characterized in that the components of the rubber/adhesive substance are dissolved in an organic solvent, and the drug is dispersed in the solution, together with the additive substance in a quantity of 3 to 30% by weight, referred to the weight of the rubber/adhesive resin substance, whereafter the dispersion, possibly after the evaporation of a proportion of the solvent, is applied to a protective layer provided with an adhesive, the solvent is substantially evaporated, a cover layer is applied, and the resulting dressing film is divided into portions of the therapeutically required size.

9. A process according to one of claims 8 to 10, characterized in that the drug is estradiol and the additive substance is a galactomannan.

10. A process according to claim 8, characterized in that the drug is bupranolol and the additive substance is microcrystalline cellulose.

11. A process according to claim 8, characterized in that the drug is nitroglycerin and the additive substance is microcrystalline cellulose.

12. A process according to claim 8, characterized in that the drug is propanolol and the additive substance is microcrystalline cellulose.

## Claims (Claims for the following Contracting State(s): AT)

1. A medicated dressing for the controlled release of drugs to the skin, comprising a covering layer, a water-insoluble adhesive film of a rubber/adhesive resin substance which is connected thereto and in which the or each drug is soluble or partially soluble and which contains an additive substance swellable in water and insoluble in the rubber/adhesive substance, and a releasable protective layer which covers the adhesive film, characterized in that the components of the rubber/adhesive substance are dissolved in an organic solvent and the drug is dispersed in the solution, together with a compound selected from the group formed by galactomannan, cellulose and tragacanth as additive substance in a quantity of 3 to 30% by weight, referred to the weight of the rubber/adhesive resin substance, whereafter the dispersion, possibly after the evaporation of a proportion of the solvent, is applied to a protective layer provided with an adhesive, the solvent is substantially evaporated, a cover layer is applied and the resulting dressing film is divided into parts of the therapeutically necessary size.

2. A process according to claim 1, characterized in that the drugs used are beta blockers, steroid hormones, calcium antagonists and cardiac-acting medicaments.

3. A process according to one of claims 1 or 2, characterized in that the rubber/adhesive resin substance is subdivided between a reservoir layer containing drug and the polymer swellable in water and an adhesive layer possibly containing drug, while provided between the reservoir layer and the adhesive layer is a separating layer which is permeable to the rubber/adhesive resin substance and drug dissolved therein, but is completely or substantially impermeable to the polymer swellable in water.

4. A process according to one of claims 1 to 3, characterized in that the drug is estradiol and the additive substance is a galactomannan.

5. A process according to one of claims 1 to 3, characterized in that the drug is bupranolol and the additive substance is microcrystalline cellulose.

6. A process according to one of claims 1 to 3, characterized in that the drug is nitroglycerin and the additive substance is microcrystalline cellulose.

7. A process according to one of claims 1 to 3, characterized in that the drug is propanolol and the additive substance is microcrystalline cellulose.

## Revendications (Revendications pour l'(les) Etat(s) contractant(s) suivant(s): BE, CH, DE, FR, GB, IT, LI, LU, NL, SE)

1. Emplâtre à matière active pour la distribution contrôlée de produits médicamenteux à la peau, constitué d'une couche de recouvrement, d'un film adhésif insoluble dans l'eau relié à elle, en une masse de caoutchouc/masse de résine adhésive dans laquelle la ou les matières actives sont solubles ou partiellement solubles, avec un additif gonflant dans l'eau et insoluble dans la masse de caoutchouc/masse adhésive, et d'une couche de protection recouvrant le film adhésif et pouvant être séparée à nouveau,
caractérisé en ce qu'on utilise un composé du groupe galactomannane, cellulose et gomme adragante en tant qu'additif en quantité de 3 à 30 % en poids rapportée au poids de la masse de caoutchouc/masse de résine adhésive.

2. Emplâtre à matière active selon la revendication 1,
caractérisé en ce qu'on utilise comme produits médicamenteux des hormones stéroïdes béta-bloquantes, des antagonistes du calcium et des médicaments cardio-actifs.

3. Emplâtre à matière active selon l'une des revendications 1 ou 2,
caractérisé en ce que la masse de caoutchouc/masse de résine adhésive est divisée en une couche-réservoir contenant une matière active et le polymère gonflant dans l'eau, et une couche auto-adhésive contenant le cas échéant une matière active, une couche de séparation étant prévue entre la couche-réservoir et la couche auto-adhésive, qui est perméable pour la masse de caoutchouc/masse de résine adhésive et la matière active dissoute dedans, totalement ou essentiellement imperméable pour le polymère gonflant dans l'eau.

4. Emplâtre en matière active pour de l'estradiol selon l'une des revendications 1 à 3,
caractérisé en ce que le polymère gonflant dans l'eau est de la galactomannane.

5. Emplâtre en matière active pour du bupranolol selon l'une des revendications 1 à 3,
caractérisé en ce que le polymère gonflant dans l'eau est de la cellulose microcristalline.

6. Emplâtre en matière active pour de la nitroglycérine selon l'une des revendications 1 à 3,
caractérisé en ce que le polymère gonflant dans l'eau est de la microcristalline.

7. Emplâtre en matière active pour du propranolol selon l'une des revendications 1 à 3,
caractérisé en ce que le polymère gonflant dans l'eau est de la cellulose microcristalline.

8. Procédé pour la fabrication d'emplâtres a matière active selon l'une des revendications 1 à 7,
caractérisé en ce qu'on dissout les parties constitutives de la masse de caoutchouc/masse adhésive dans un solvant organique et on disperse la matière active dans la solution, en même temps que l'additif en quantité de 3 à 30 % en poids rapportée au poids de la masse de caoutchouc/masse de résine adhésive, on applique la dispersion, le cas échéant après évaporation d'une partie du solvant, sur une couche de protection équipée d'adhésif, on évapore sensiblement le solvant, on applique une couche de recouvrement et on divise le film-emplâtre ainsi obtenu en morceaux de grandeur utilisable thérapeutiquement.

9. Procédé selon la revendication 8,
caractérisé en ce que la matière active est de l'estradiol et l'additif de la galactomannane.

10. Procédé selon la revendication 8,
caractérisé en ce que la matière active est du bupranolol et l'additif de la cellulose microcristalline.

11. Procédé selon la revendication 8,
caractérisé en ce que la matière active est de la nitroglycérine et l'additif de la cellulose microcristalline.

12. Procédé selon la revendication 8,
caractérisé en ce que la matière active est du propanolol et l'additif de la cellulose microcristalline.

## Revendications (Revendications pour l'(les) Etat(s) contractant(s) suivant(s): AT)

1. Procédé pour la fabrication d'emplâtres à matière active pour la distribution contrôlée de produits médicamenteux à la peau, constitués d'une couche de recouvrement, d'un film adhésif insoluble dans l'eau relié à elle, en une masse de caoutchouc/masse de résine adhésive, dans laquelle la ou les matières actives est ou respectivement sont soluble(s) ou partiellement soluble(s), avec un additif gonflant dans l'eau et insoluble dans la masse de caoutchouc/masse adhésive, et d'une couche de protection recouvrant le film adhésif et pouvant être séparée à nouveau,
caractérise en ce qu'on dissout les parties constitutives de la masse de caoutchouc/masse adhésive dans un solvant organique et on disperse la matière active dans la solution en même temps qu'un composé du groupe galactomannane, cellulose et gomme adragante en tant qu'additif en quantité de 3 à 30 % en poids rapportée au poids de la masse de caoutchouc/masse de résine adhésive, on applique la dispersion, le cas échéant après évaporation d'une partie du solvant, sur une couche de protection équipée d'adhésif, on évapore sensiblement le solvant, on applique une couche de recouvrement et on divise le film-emplâtre ainsi obtenu en morceaux de grandeur utilisable thérapeutiquement.

2. Procédé selon la revendication 1,
caractérisé en ce qu'on utilise comme produits médicamenteux des hormones stéroïdes béta-bloquantes, des antagonistes du calcium et des médicaments cardio-actifs.

3. Procédé selon l'une des revendications 1 ou 2,
caractérisé en ce qu'on divise la masse de caoutchouc/masse de résine adhésive en une couche-réservoir contenant une matière active et l'additif et une couche auto-adhésive contenant le cas échéant une matière active, une couche de séparation étant prévue entre la couche-réservoir et la couche auto-adhésive, qui est perméable pour la masse de caoutchouc/masse de résine adhésive et la matière active dissoute dedans, totalement ou essentiellement imperméable pour l'additif.

4. Procédé selon l'une des revendications 1 à 3,
caractérisé en ce que la matière active est de l'estradiol et l'additif de la galactomannane.

5. Procédé selon l'une des revendications 1 à 3,
caractérisé en ce que la matière active est du bupranolol et l'additif de la cellulose microcristalline.

6. Procédé selon l'une des revendications 1 à 3,
caractérisé en ce que la matière active est de la nitroglycérine et l'additif de la cellulose microcristalline.

7. Procédé selon l'une des revendications 1 à 3,
caractérisé en ce que la matière active est du propanolol et l'additif de la cellulose microcristalline.
